# EUROPEAN PATENT APPLICATION

(11) **EP 4 265 295 A1**
(43) Date of publication of application: **25.10.2023**
(21) Application number: 22171131.0
(22) Date of filing: 02.05.2022
(51) Int. Cl.: A61N 5/06, A61B 18/00

(54) **TREATMENT AND MONITORING OF TISSUE REPERFUSION AND ASSOCIATED DEVICES, SYSTEMS, AND METHODS**

(30) Priority: 20.04.2022 US 202263332722 P
(71) Applicant: Koninklijke Philips N.V., 5656 AG Eindhoven (NL)
(72) Inventor: FRANCIS, Nathan C., Eindhoven (NL)
(74) Representative: Philips Intellectual Property & Standards

(57) **Abstract**

An apparatus (402) includes a flexible elongate member (422) configured to be positioned within a body lumen (420) of a patient. The apparatus includes at least one light emitter (506a) positioned within the flexible elongate member. The at least one light emitter is configured to emit light into tissue of the patient. The apparatus includes at least one light detector (508) positioned within the flexible elongate member. The at least one light detector is configured to receive light from the tissue, indicative of an absorption of the light within the tissue of the patient.

## Description

### TECHNICAL FIELD

The subject matter described herein relates to reperfusion therapy, and, in particular, to the treatment and monitoring of tissue reperfusion.

### INTRODUCTION

A percutaneous coronary intervention (PCI) may be utilized to treat a blockage (e.g., an occlusion, a lesion, a stenosis, and/or the like) within a blood vessel. The PCI may include a therapeutic procedure, such as administration of a drug, angioplasty, placement of a stent, and/or the like, that reduces a size of the blockage or opens (e.g., widens) the lumen of the affected blood vessel. To that end, PCI may restore blood flow through a blood vessel and to tissue that receives blood/oxygen via the blood vessel. Moreover, before a PCI therapy is delivered, the reduction in blood flow caused by a blockage within a blood vessel may cause tissue that receives blood/oxygen from the vessel to experience ischemia. Accordingly, PCI may restore or increase blood flow to tissue that experienced ischemia, which may restore the health of the tissue. However, even after a PCI therapy is delivered, blood/oxygen may not always suitably re-perfuse through tissue that has experienced ischemia. In particular, the increase and/or reintroduction of blood flow through the ischemic tissue may trigger an inflammatory response and/or oxidative damage, known as reperfusion injury, along with or in place of restoration of normal function of the tissue.

In body lumens, such as a coronary artery of a chamber of the heart where, for example, a myocardial infarction, or heart attack, occurs when the myocardium (heart muscle) is starved of blood and oxygen, usually from a blockage in the coronary arteries, the myocardium is damaged during the infarction. If the blockage is cleared quickly enough and oxygenated blood may perfuse the injured site, the myocardium may remodel back to healthy tissue. If the injured myocardium doesn't receive blood flow, the myocardial tissue dies and the area fibroses, eliminated that contractile segment from the heart. This makes pumping of the heart less efficient, which may affect quality of life for the patient.

The information included in this Introduction section of the specification, including any references cited herein and any description or discussion thereof, is included for context and/or technical reference purposes only and is not to be regarded as subject matter by which the scope of the disclosure is to be bound or otherwise limited in any manner.

### SUMMARY

Disclosed are catheter-based light delivery devices, systems, and methods that utilize an array of light emitters and detectors to both treat an ischemic area and monitor the reperfusion of the treated area. For example, an intraluminal device may include an array of light emitters and detectors to treat the ischemic area and monitor the reperfusion of the treated area.

In some instances, the disclosed apparatus delivers light having a wavelength of approximately 670 nm light to infarcted areas to trigger release of nitric oxide from the tissue and/or otherwise stimulate reperfusion of tissue. In some instance, the delivered light may also be used to monitor oxygenation of tissue, using the tissue/blood as an absorption source. A detector monitors an absorption of the light within the tissue of the patient, which indicates a level of oxygenation of the tissue. Nitric oxide is a natural compound in blood vessels that act as a vasodilator, expanding the diameter of blood arteries to allow more blood flow. It has been shown that 670 nanometer (nm) light may trigger release of nitric oxide stores within the body to increase perfusion to different tissue areas. These nitric oxide stores come from the blood itself and from vessel walls. It has also been demonstrated that the dilating effect of nitric oxide increases ability of damaged tissue to repair itself faster with greater influx of oxygenated blood.

The aspects and features disclosed herein have particular, but not exclusive, utility for intraluminal medical catheters and/or guidewires. In some instances, an apparatus in accordance with the present disclosure comprises: a flexible elongate member configured to be positioned within a body lumen of a patient; at least one light source positioned within the flexible elongate member, the at least one light source configured to emit light into tissue of the patient; and at least one light detector positioned within the flexible elongate member, the at least one light detector configured to monitor an absorption of the light within the tissue of the patient.

In an exemplary aspect, an apparatus is provided. The apparatus includes a flexible elongate member configured to be positioned within a body lumen of a patient; at least one light emitter positioned within the flexible elongate member, the at least one light emitter configured to emit light into tissue of the patient; and at least one light detector positioned within the flexible elongate member, the at least one light detector configured to monitor an absorption of the light within the tissue of the patient.

In some aspects, the absorption of the light within the tissue of the patient indicates a level of oxygenation of the tissue. In some aspects, the at least one light emitter is positioned within a distal portion of the flexible elongate member. In some aspects, the at least one light detector is positioned within the distal portion of the flexible elongate member. In some aspects, the distal portion of the flexible elongate member is configured to be positioned in contact with the tissue of the patient. In some aspects, the at least one light emitter includes a plurality of light emitters arranged in a linear array. In some aspects, the at least one light detector includes a plurality of light detectors arranged in a linear array. In some aspects, the at least one light emitter includes a plurality of light emitters arranged in a matrix array. In some aspects, the at least one light detector includes a plurality of light detectors arranged in a matrix array. In some aspects, the at least one light emitter is configured to emit the light having a wavelength of approximately 670-nanometers. In some aspects, the at least one light detector is a plurality of light detectors, wherein one or more of the plurality of light detectors are offset from the at least one light emitter in a direction along or parallel to a longitudinal axis of the flexible elongate member. In some aspects, the at least one light detector is a plurality of light detectors, wherein one or more of the plurality of light detectors are offset from the at least one light emitter in a direction perpendicular to a longitudinal axis of the flexible elongate member. In some aspects, the at least one light emitter is a plurality of light emitters, wherein one of more of the plurality of light emitters are offset from the at least one light detector in a direction along or parallel to a longitudinal axis of the flexible elongate member. In some aspects, the at least one light emitter is a plurality of light emitters, wherein one of more of the plurality of light emitters are offset from the at least one light detector in a direction perpendicular to a longitudinal axis of the flexible elongate member. In some aspects, the apparatus further includes a processing system configured to be in communication with the at least one light emitter and the at least one light detector, wherein the processing system is configured to: control a timing of emission of the light from the at least one light emitter; and control a timing of detection of light by the at least one light detector to monitor the absorption of the light within the tissue. In some aspects, the apparatus further includes a display in communication with the processing system, wherein the processing system is configured to output a visual representation of the absorption of the light within the tissue via the display.

In an exemplary aspect, a method is provided. The method includes introducing a flexible elongate member into a body lumen of a patient, wherein the flexible elongate member includes at least one optical fiber; emitting, by at least one light emitter positioned within the flexible elongate member, light into tissue of the patient; and monitoring, by at least one light detector positioned within the flexible elongate member, an absorption of the light within the tissue of the patient.

This Summary is provided to introduce a selection of concepts in a simplified form that are further described below in the Detailed Description. This Summary is not intended to identify key features or essential features of the claimed subject matter, nor is it intended to limit the scope of the claimed subject matter. A more extensive presentation of features, details, utilities, and advantages of the metal ink conductor assembly, as defined in the claims, is provided in the following written description of various examples and/or aspects of the disclosure and illustrated in the accompanying drawings.

### BRIEF DESCRIPTION OF THE DRAWINGS

Illustrative aspects of the present disclosure will be described with reference to the accompanying drawings, of which:
Fig. 1 is a diagrammatic, schematic view of a system according to aspects of the present disclosure.
Fig. 2 is a schematic diagram of a processing system according to aspects of the present disclosure.
Fig. 3A is diagram of a human heart with an obstruction according to aspects of the present disclosure.
Fig. 3B is diagram of the human heart following a percutaneous coronary intervention (PCI) according to aspects of the present disclosure.
Fig. 3C is diagram of the human heart following a reperfusion therapy according to aspects of the present disclosure.
Fig. 4 is a diagrammatic schematic view of an intraluminal system 400 according to aspects of the present disclosure.
Figs. 5A and 5B are diagrammatic side views of an array of a flexible elongate member within a body lumen according to aspects of the present disclosure.
Figs. 6A and 6B are diagrammatic side views of an array of a flexible elongate member within a body lumen according to aspects of the present disclosure.
Figs. 7A and 7B are diagrammatic side views of an array of a flexible elongate member within a body lumen according to aspects of the present disclosure.
Figs. 8A and 8B are diagrammatic side views of an array of a flexible elongate member within a body lumen according to aspects of the present disclosure.
Figs. 9A and 9B are diagrammatic side views of an array of a flexible elongate member within a body lumen according to aspects of the present disclosure.
Figs. 10A and 10B are diagrammatic side views of an array of a flexible elongate member within a body lumen according to aspects of the present disclosure.
Fig. 11 is an exemplary illustration of resolved depth information from the light emitted from a plurality of light emitters as detected by a light detector according to aspects of the present disclosure.

### DETAILED DESCRIPTION

For the purposes of promoting an understanding of the principles of the present disclosure, reference will now be made to the examples illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one example and/or aspect may be combined with the features, components, and/or steps described with respect to other examples and/or aspects of the present disclosure. Additionally, while the description below may refer to blood vessels, it will be understood that the present disclosure is not limited to such applications. For example, the devices, systems, and methods described herein may be used in any body chamber or body lumen, including an esophagus, veins, arteries, intestines, ventricles, atria, or any other body lumen and/or chamber. For the sake of brevity, however, the numerous iterations of these combinations will not be described separately.

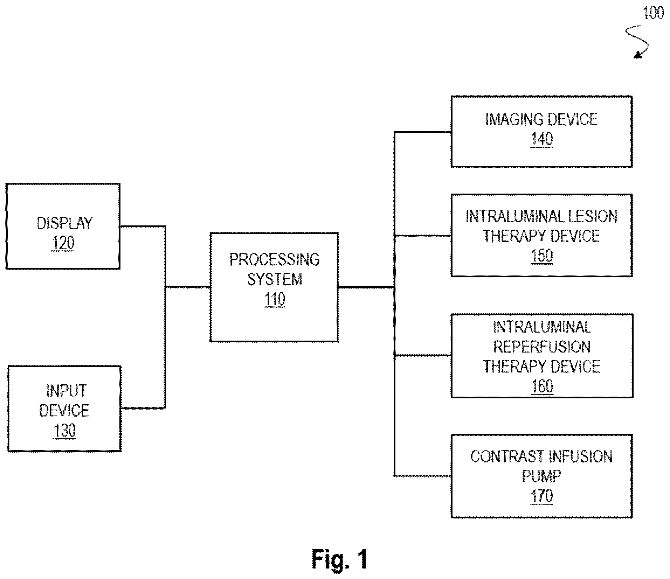

Referring to Fig. 1, shown is a diagrammatic, schematic view of a system according to aspects of the present disclosure. The system 100 may be configured to evaluate (e.g., assess), display, and/or control (e.g., modify) one or more aspects of a reperfusion therapy targeting an area of a patient's body, such as a portion of the myocardium. For instance, the system 100 may be utilized to monitor and/or control reperfusion therapy such that injury to the myocardium following a percutaneous coronary intervention (PCI) is avoided or minimized, as described in greater detail below. In this regard, the system 100 may be used to assess coronary vessels and/or heart tissue (e.g., the myocardium) oxygenated by the coronary vessels. As illustrated, the system 100 may include a processing system 110 in communication with a display device 120 (e.g., an electronic display or monitor), an input device 130 (e.g., a user input device, such as a keyboard, mouse, joystick, microphone, and/or other controller or input device), an imaging device 140, an intravascular lesion therapy device 150 (e.g., intraluminal therapy device), an intravascular reperfusion therapy device 160 (e.g., intraluminal reperfusion therapy device), and/or a contrast infusion pump 170.

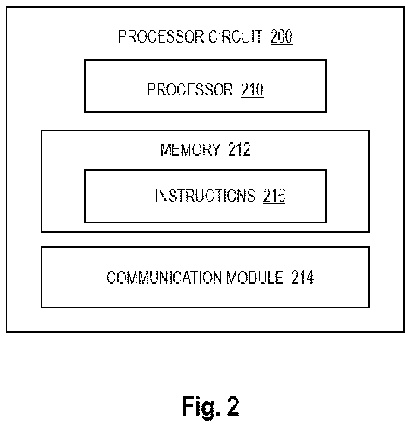

The processing system 110 is generally representative of any device suitable for performing the processing and analysis techniques disclosed herein. In some aspects, the processing system 110 includes a processor circuit, such as the processor circuit 200 of Fig. 2. In some aspects, the processing system 110 is programmed to execute steps associated with the data acquisition, analysis, and/or instrument (e.g., device) control described herein. Accordingly, it is understood that any steps related to data acquisition, data processing, instrument control, and/or other processing or control aspects of the present disclosure may be implemented by the processing system 110 (e.g., computing device) using corresponding instructions stored on or in a non-transitory computer readable medium accessible by the computing device. In some instances, the processing system 110 is a console device. Further, it is understood that in some instances the processing system 110 comprises one or a plurality of computing devices, such as computers, with one or a plurality of processor circuits. In this regard, it is particularly understood that the different processing and/or control aspects of the present disclosure may be implemented separately or within predefined groupings using a plurality of computing devices. Any divisions and/or combinations of the processing and/or control aspects described below across multiple computing devices are within the scope of the present disclosure.

Fig. 2 is a schematic diagram of a processing system according to aspects of the present disclosure. The processor circuit 200 may be implemented in and/or as part of the processing system 110 of Fig. 1. As shown, the processor circuit 200 may include a processor 210, a memory 212, and a communication module 214. These elements may be in direct or indirect communication with each other, for example via one or more buses. The processor 210 may include a central processing unit (CPU), a digital signal processor (DSP), an ASIC, a controller, an FPGA, another hardware device, a firmware device, or any combination thereof configured to perform the operations described herein. The processor 210 may also be implemented as a combination of computing devices, e.g., a combination of a DSP and a microprocessor, a plurality of microprocessors, one or more microprocessors in conjunction with a DSP core, or any other such configuration.

The memory 212 may include a cache memory (e.g., a cache memory of the processor 210), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In some instances, the memory 212 includes a non-transitory computer-readable medium. The memory 212 may store instructions 216. The instructions 216 may include instructions that, when executed by the processor 210, cause the processor 210 to perform the operations described herein with reference to the processing system 110 (Fig. 1). Instructions 216 may also be referred to as code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements.

The communication module 214 may include any electronic circuitry and/or logic circuitry to facilitate direct or indirect communication of data between various components of the processor circuit 200 and/or the processing system 110 (Fig. 1). Additionally or alternatively, the communication module 214 may facilitate communication of data between the processor circuit 200, the display device 120, the input device 130, the imaging device 140, the intravascular lesion therapy device 150, the intravascular reperfusion therapy device 160, the contrast infusion pump 170, and/or the like. In this regard, the communication module 214 may be an input/output (I/O) device interface, which may facilitate communicative coupling between the processor circuit 200 and (I/O) devices, such as the input device 130. Moreover, the communication module 214 may facilitate wireless and/or wired communication between various elements of the processor circuit 200 and/or the devices and systems of the system 100 using any suitable communication technology, such as a cable interface such as a USB, micro-USB, Lightning, or FireWire interface, Bluetooth, Wi-Fi, ZigBee, Li-Fi, or cellular data connections such as 2G/GSM, 3G/UMTS, 4G/LTE/WiMax, or 5G.

Turning back now to Fig. 1, the imaging device 140 may include an x-ray system, angiography system, fluoroscopy system, ultrasound system, computed tomography (CT) system, a magnetic resonance imaging (MRI) system, other suitable imaging devices, and/or combinations thereof. The imaging device 140 may additionally or alternatively include a nuclear medicine imaging device, such as a gamma camera or a single-photon emission computed tomography (SPECT) system, other suitable devices, and/or combinations thereof. In some aspects, the imaging device 140 may be configured to acquire imaging data of anatomy, such as the heart and blood vessels, while the imaging device 140 is positioned outside of the body of the patient. The imaging data may be visualized in the form of two-dimensional and/or three-dimensional images of the heart, blood vessel, and/or other anatomy. In some aspects, the imaging device 140 may be an internal device that is positioned inside the patient body. For example, the imaging device 140 may be an intracardiac echocardiography (ICE) catheter that obtains images while positioned within a heart chamber. In some aspects, the imaging device 140 may be an external device in that is it is positioned outside of the particular anatomy that is being imaged (e.g., blood vessels and/or heart), but is positioned inside the patient body. For example, the imaging device 140 may be a transesophageal echocardiography (TEE) probe that obtains images while positioned within an esophagus.

Moreover, the imaging device 140 may obtain images of the heart that are indicative of the health of the cardiac muscle or myocardium. In particular, the imaging device 140 may be configured to acquire imaging data that illustrates myocardial perfusion (e.g., myocardial perfusion imaging (MPI) data). For example, MPI data may be collected by imaging a radiopharmaceutical agent, such as thallium, in the patient's heart muscle using a SPECT system. Additionally or alternatively, the imaging data may be obtained by imaging a contrast agent, which may be administered to the patient's vasculature manually or via the contrast infusion pump 170, for example. In any case, the imaging data may illustrate vasculature and/or muscle mass with blood flow and/or vasculature and/or muscle mass that lack of blood flow in areas of the heart.

The contrast infusion pump 170 may administer a contrast agent that may alter an appearance (e.g., a brightness, an intensity, a contrast) of a feature within imaging data, such as the imaging data obtained by the imaging device 140. In this regard, the contrast infusion pump 170 may be configured to administer, to the patient, a contrast agent that is radiopaque and enhances the visibility of internal fluids or structures within a patient's anatomy. In some aspects, for example, the contrast agent absorbs external x-rays from an x-ray source, resulting in decreased exposure on an x-ray detector in conjunction with the x-ray source. The contrast agent may be of any suitable material, chemical, or compound and, before administration to the patient, may be a liquid, powder, paste, tablet, or of any other suitable form. For example, the contrast agent may include iodine-based compounds, barium sulfate compounds, gadolinium-based compounds, microbubbles, or any other suitable compounds, which may be included in a solution or suspension, for example, for administration to the patient. In some aspects, the contrast agent may include carbon dioxide, which may be a gas. In such cases, the contrast agent may decrease absorption of the external x-rays from the x-ray source, when administered. The contrast agent may additionally be referred to as a radiocontrast agent, a contrast dye, a radiocontrast dye, a contrast material, a radiocontrast material, a contrast media, or a radiocontrast media, among other terms. Further, in some aspects, the contrast infusion pump 170 may be configured to combine or switch between different contrast agents, which may reduce stress on the patient's body. For instance, the contrast infusion pump 170 may administer a first contrast agent for a period of time and may subsequently administer a different, second contrast agent to the patient during an imaging procedure.

The intravascular lesion therapy device 150 may be any form of device, instrument, or probe sized and shaped to be positioned within a vessel. For example, the intravascular lesion therapy device 150 is generally representative of a guide wire, a catheter, or a guide catheter. However, in other aspects, the intravascular lesion therapy device 150 may take other forms. In this regard, the intravascular lesion therapy device 150 may be a device configured to deliver a PCI therapy to a vessel. In particular, the intravascular lesion therapy device 150 may be an intravascular guidewire or catheter configured to ablate a lesion (e.g., a blockage) within the vessel, deploy a balloon, a stent, and/or drug to a target site within the vessel, and/or the like. That is, for example, the intravascular lesion therapy device 150 may be a stent or balloon delivery device (e.g., an angioplasty device), a thrombectomy device, an atherectomy device, and/or the like. In this regard, the intravascular lesion therapy device 150 may include a coil retriever, an aspiration (e.g., suction) device, and/or the like to assist in the removal of a clot or occlusion from the patient's vessel. In some aspects, the intravascular lesion therapy device 150 may include a laser, a blade (e.g., knife), a sanding crown, and/or any suitable device that may assist in the cutting, shaving, sanding, vaporizing, and/or removal of atherosclerotic plaque from the patient's vessel. Additionally or alternatively, the intravascular lesion therapy device 150 may be the therapy itself delivered to the vessel. More specifically, the intravascular lesion therapy device 150 may represent a stent or balloon deployed to the vessel, a drug administered intra or extravascularly (e.g., orally), and/or the like. To that end, while the intravascular lesion therapy device 150 is illustrated as being communicatively coupled to the processing system 110, aspects are not limited thereto.

In some aspects, the intravascular reperfusion therapy device 160 may be a device, instrument, or probe sized and shaped to be positioned within a vessel. In accordance with the present disclosure, the intravascular reperfusion therapy device 160 may include an intraluminal device having one or more side-emitting optical fibers. In some instances, the intravascular reperfusion therapy device 160 may be a device or instrument configured to control reperfusion of blood flow into a target tissue area (e.g., capillary bed), such as a portion of the myocardium of a patient. In some aspects, the target tissue area may be an ischemic area and/or an area of tissue that receives reduced blood flow due to a blockage in an associated vessel (e.g., an upstream artery). As described in greater detail below, treatment (e.g., therapy) directed to the vessel associated with the blockage, such as treatment via the intravascular lesion therapy device 150, may reintroduce or increase blood flow to the target tissue area. To reduce and/or prevent injury to the target tissue area resulting from this increased blood flow, the intravascular reperfusion therapy device 160 may be positioned intravascularly, such as within a coronary blood vessel, and may be configured to direct light into and/or around the target tissue area. In some instances, the light directed into and/or around the target tissue area may have a wavelength configured to stimulate the release of nitric oxide (NO). In some aspects, the reperfusion therapy may also include administration of anti-inflammatory drug(s) or nitric oxide (NO) to the patient. In some aspects, the reperfusion therapy may include cold fluid that is provided via the arterial side.

In some aspects, one or more of the imaging device 140, the intravascular lesion therapy device 150, the intravascular reperfusion therapy device 160, and/or the contrast infusion pump 170, are located proximate one or more of the processing system 110, the display device 120, and/or the input device 130, such as in the same procedure room. In some aspects, one or more of the imaging device 140, the intravascular lesion therapy device 150, the intravascular reperfusion therapy device 160, and/or the contrast infusion pump 170 are located spaced from one or more of the processing system 110, the display device 120, and/or the input device 130, such as in different procedure rooms or facilities. For example, the imaging device 140, the intravascular lesion therapy device 150, the intravascular reperfusion therapy device 160, and/or the contrast infusion pump 170 may be part of different systems that are communicatively coupled. In this regard, the processing system 110 may be configured to acquire the data collected from the components spaced therefrom and process the data as described herein. The imaging device 140, the intravascular lesion therapy device 150, the intravascular reperfusion therapy device 160, and/or the contrast infusion pump 170 may be configured to transmit the collected data to the processing system 110.

The system 100 includes a display device 120 that is communicatively coupled to the processing system 110. In some aspects, the display device 120 is a component of the processing system 110, while in other aspects, the display device 120 is distinct from the processing system 110. In some aspects, the display device 120 is a monitor integrated in a console device or a standalone monitor (e.g., a flat panel or flat screen monitor). The processing system 110 may be configured to generate a visual display (e.g., screen display) based on imaging data from the imaging device 140. The processing system 110 may provide (e.g., output) the screen display to the display device 120. To that end, the display device 120 may be configured to output (e.g., display) a two-dimensional image and/or a two-dimensional representation of the heart, blood vessels, and/or other anatomy, which may be included in the screen display. In some aspects, the display device 120 is configured to output a three-dimensional graphical representation of the heart, blood vessels, and/or other anatomy. For instance, the display device 120 may be a holographic display device configured to output a three-dimensional holographic display of anatomy. Any suitable display device is within the scope of this disclosure, including self-contained monitors, projection/screen systems, head-up display systems, etc. The display device may implement principles based on moving reflective microelectromechanical systems (MEMS), laser plasma, electro-holography, etc. In some aspects, the display device 120 is implemented as a bedside controller having a touch-screen display as described, for example, in U.S. Provisional Application No. 62/049,265, titled "Bedside Controller for Assessment of Vessels and Associated Devices, Systems, and Methods," and filed September 11, 2014, the entirety of which is hereby incorporated by reference herein.

The system 100 includes an input device 130 that is communicatively coupled to the processing system 110. The input device 130 may be a peripheral device, such as a touch sensitive pad, a touch-screen, a joy-stick, a keyboard, mouse, trackball, a microphone, an imaging device, and/or the like. In other aspects, the user interface device is part of the display device 120, which may be a touch-screen display, for example. Moreover, a user may provide an input to the processing system 110 via the input device 130. In particular, the input device 130 may enable a user to control, via inputs to the processing system 110, one or more of the components of the system 100, such as the imaging device 140, the intravascular lesion therapy device 150, the intravascular reperfusion therapy device 160, the contrast infusion pump 170, or the processing system 110 itself. Additionally or alternatively, the input device 130 may facilitate interaction with a screen display provided at the display device 120. For instance, a user may select, edit, view, or interact with portions of the screen display (e.g., a GUI) provided at the display device 120 via the input device 130.

The system 100 may include various connectors, cables, interfaces, connections, etc., to communicate between the elements of the intravascular lesion therapy device 150, the intravascular reperfusion therapy device 160, the processing system 110, the imaging device 140, the display device 120, and/or the input device 130. In some aspects, for example, the communication module 214 (Fig. 2), which may be included in the processing system 110, may include such connectors, interfaces, and/or the like. In this regard, the processing system 110 may communicate and/or control one or more components of the processing system 110 via mechanical and/or electromechanical signaling and/or controls. Further, the illustrated communication pathways are exemplary in nature and should not be considered limiting in any way. In this regard, it is understood that any communication pathway between the components of system 100 may be utilized, including physical connections (including electrical, optical, and/or fluid connections), wireless connections, and/or combinations thereof. In this regard, it is understood that the one or more of the components of the system 100 may communicate via a wireless connection in some instances. In some instances, the one or more components of the system 100 and/or other systems (e.g., of a hospital or health services provider) communicate via a communication link over a network (e.g., intranet, internet, telecommunications network, and/or other network).

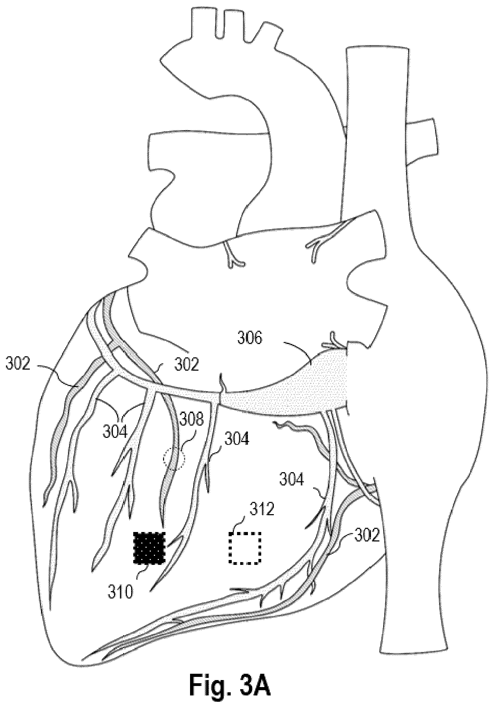

Figs. 3A-3C illustrate a diagram of a human heart 300. As illustrated, the heart 300 includes coronary arteries 302 (illustrated with a first fill pattern) that deliver oxygenated blood to tissue, such as muscle tissue (e.g., myocardium), of the heart 300. The heart 300 further includes coronary veins 304 (illustrated with a second fill pattern), including a coronary sinus 306, that carry deoxygenated blood away from the tissue of the heart and towards a chamber (e.g., an atrium) of the heart 300.

In the diagram illustrated in Fig. 3A, a coronary artery 302 of the heart 300 includes a blockage 308 (e.g., an occlusion, a lesion, a stenosis, and/or the like) according to aspects of the present disclosure. The blockage 308 may disrupt flow through the coronary artery 302. In particular, the blockage 308 may decrease the diameter of a portion of the lumen of the coronary artery 302, which may decrease the flow of blood through the portion of the lumen. As a result, a first area of tissue 310 (e.g., a portion of the myocardium) that is associated with (e.g., receives blood from) the coronary artery 302 with the blockage 308 may not receive a healthy amount of blood/oxygen. For instance, the blood/oxygen delivered to the first area of tissue 310 may not be sufficient to perfuse through (e.g., to be distributed across) the entire first area of tissue 310 in some cases. In this regard, the first area of tissue 310 may experience ischemia (e.g., a reduction in delivered blood/oxygen illustrated by a fill pattern), which may damage the first area of tissue 310. The illustrated different, second area of tissue 312 (e.g., a portion of the myocardium) may receive blood/oxygen from a different coronary artery 302 than the first area of tissue 310. In this regard, the second area of tissue 312 may remain relatively unaffected by the blockage 308. To that end, the second area of tissue 312 may receive a healthy amount of blood/oxygen, and the second area of tissue 312 may not experience ischemia. Accordingly, the second area of tissue 312 is illustrated as being healthy by a lack of the fill pattern shown in the first area of tissue 310.

In some aspects, a percutaneous coronary intervention (PCI) may be utilized to treat the blockage 308. In particular, the PCI may include a therapeutic procedure that reduces a size of the blockage 308, opens (e.g., widens) the lumen of a vessel, and/or the like to restore blood flow through the vessel (e.g., the coronary artery 302) with the blockage 308. In this regard, the PCI may include, for example, angioplasty (e.g., deploying a balloon) and positioning a stent across the stenosis to open the vessel (e.g., the coronary artery 302 with the blockage). The PCI may additionally or alternatively include thrombectomy, atherectomy, administration of a drug and/or the like. To that end, the intravascular lesion therapy device 150 (Fig. 1) may facilitate and/or provide the PCI to a vessel having a blockage (e.g., blockage 308).

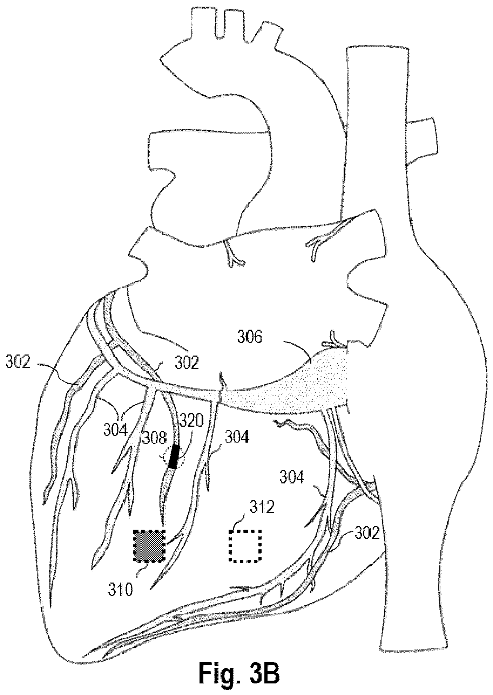

Fig. 3B illustrates a diagram of the heart 300 after delivery of a therapeutic procedure (e.g., post-treatment), such as PCI according to aspects of the present disclosure. In particular, Fig. 3B illustrates a stent 320 positioned within the coronary vessel at the site of the blockage 308. However, the various aspects are not limited thereto. In this regard, the PCI delivered to the coronary artery 302 or a vessel with a blockage may include any suitable combination of the therapies described above.

As described above, the stent 320 and/or another suitable PCI (e.g., therapeutic procedure) may be provided to a vessel so that an effect of a blockage on blood flow through the vessel is reduced. In this regard, the placement of the stent 320 within the coronary artery 302 (e.g., at the site of the blockage 308) may open (e.g., widen) the portion of the lumen of the coronary artery 302 with the blockage 308, which may increase blood flow through the portion lumen. Moreover, the placement of the stent 320 within the heart 300 may increase blood flow downstream of the blockage 308, such as within vasculature that receives blood flow from the portion of the lumen. In this way, the vasculature (e.g., a capillary bed) that delivers blood/oxygen to the first area of tissue 310 may receive increased blood flow, which may increase blood/oxygen delivery to the first area of the tissue 310. To that end, blood/oxygen may re-perfuse the first area of the tissue 310. Accordingly, the stent 320 may reverse or reduce the ischemia experienced by the first area of the tissue 310. In this regard, the first area of the tissue 310 is illustrated in Fig. 3B with a different fill pattern than the fill pattern illustrated in Fig. 3A to demonstrate the increased blood/oxygen supplied to the first area of the tissue 310.

In some cases, blood/oxygen may not suitably perfuse through tissue associated with an occluded vessel (e.g., a vessel with a blockage), such as the first area of tissue 310, after delivery of a PCI therapy. For example, in some cases, the introduction and/or increase of blood flow to tissue that has experienced ischemia may result in reperfusion injury (e.g., ischemia-reperfusion injury). In particular, the returned blood flow may trigger an inflammatory response and/or oxidative damage along with or in place of restoration of normal function of the tissue. Inflammation, damage resulting from inflammation, and/or the oxidative damage may obstruct the flow of blood/oxygen within the tissue (e.g., within a capillary bed associated with the tissue). Accordingly, blood/oxygen may not be distributed throughout (e.g., perfuse through) the tissue at a healthy level even after the delivery of a PCI therapy. For instance, blood may preferentially flow through a first portion of the tissue lacking inflammation and/or damage and may flow through a second portion of the tissue with inflammation and/or damage to a lesser degree. As a result, the second portion of the tissue may continue to receive blood flow below a healthy level. In this regard, the first area of the tissue 310 is illustrated in Fig. 3B with a different fill pattern than the second area of the tissue 312 (e.g., a healthy area of tissue) to demonstrate that the stent 320 alone may not fully restore the health and/or functioning of the first area of the tissue 310.

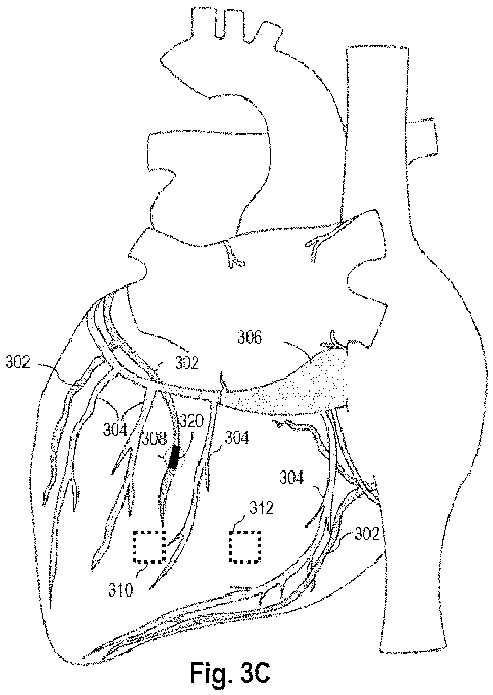

Turning now to Fig. 3C, in some cases, the reperfusion of blood/oxygen within ischemic tissue may be further assisted and/or controlled by reperfusion therapy according to aspects of the present disclosure. To that end, Fig. 3C illustrates a diagram of the heart 300 after delivery of the PCI therapy and a reperfusion therapy, such as a therapy delivered by the intravascular reperfusion therapy device 160 in accordance with the present disclosure. In particular, Fig. 3C illustrates a diagram of the heart 300 following delivery of a reperfusion therapy targeting the first area of tissue 310. In accordance with the present disclosure, one or more side-emitting optical fibers of the intravascular reperfusion therapy device 160 may be utilized to direct light into and/or around the target tissue area (e.g., the first area of tissue 310). In some instances, the light directed into and/or around the target tissue area may have a wavelength configured to stimulate the release of nitric oxide (NO), including without limitation light having a wavelength of approximately 670 nm.

According to techniques described in greater detail below, reperfusion therapy may be delivered by the intravascular reperfusion therapy device 160 to reduce or minimize injury at and/or to improve blood flow to tissue where blood is re-perfusing (e.g., an area of tissue receiving an increase in blood flow), such as the first area of tissue 310. In particular, reperfusion therapy may affect a distribution of blood flow through the targeted tissue such that blood flow perfuses (e.g., distributes to) and/or increases throughout the tissue, including through areas of the tissue that are inflamed or have oxidative damage. Accordingly, delivery of the reperfusion therapy targeting an area of tissue may restore blood flow to a healthy amount or an amount exceeding the blood flow resulting from the PCI therapy alone. For instance, in the illustrated aspects, the health and/or functioning of (e.g., the blood flow to) the first area of tissue 310 is shown as being fully restored by the reperfusion therapy (e.g., directing light into and/or around the first area of tissue 310 using the intravascular reperfusion therapy device 160), as indicated by the fill pattern of the first area of tissue 310 matching the second area of tissue 312. In some aspects, however, the reperfusion therapy may restore the health and/or functioning of (e.g., the blood flow to) tissue to a level greater than a level resulting from the PCI but less than a level at an area of tissue, such as the second area of tissue 312, that was relatively unaffected by a blockage (e.g., associated with a different vessel than the vessel having the blockage). Particular mechanisms for controlling, using one or more components of the system 100, the reperfusion of an area of tissue associated with (e.g., configured to receive blood/oxygen from) a vessel that receives a PCI therapy (e.g., a vessel that has an occlusion) and/or otherwise receives an increase in blood flow are described herein.

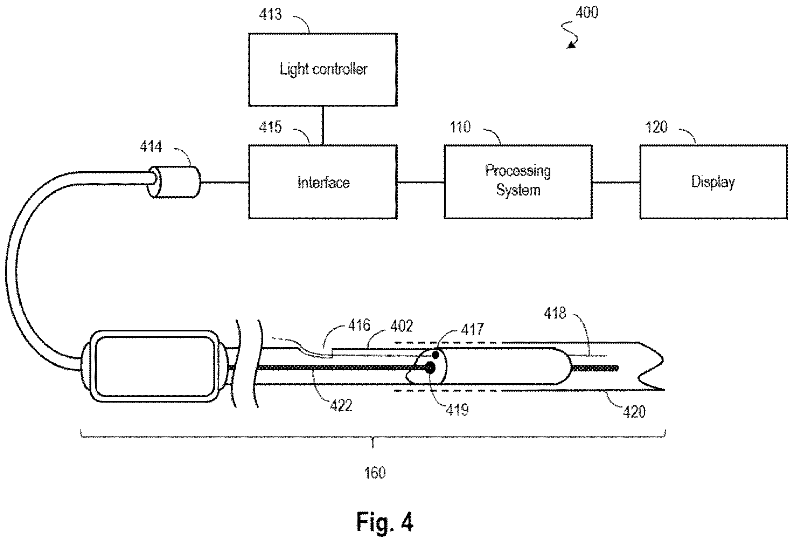

Fig. 4 is a diagrammatic schematic view of an intraluminal system 400 according to aspects of the present disclosure. The intraluminal system 400 may include an intraluminal device 402, which may be the intravascular reperfusion therapy device 160 (e.g., intraluminal reperfusion therapy device), such as a catheter, guide wire, or guide catheter, an interface 415, a processing system or controller 110, and a display device 120. At a high level, in some aspects, the intraluminal device 402 may include a flexible elongate member 422. In some instances, the flexible elongate member 422 emits light radially outward from a distal portion of the intraluminal device and into tissue, e.g., the region of interest, of a body lumen of a patient to trigger release of nitric oxide from the tissue. At a high level, the light delivered by the flexible elongate member 422 may be used to monitor oxygenation of tissue, using the tissue/blood as an absorption source. The monitored absorption of the light within the tissue of the patient indicated a level of oxygenation of the tissue. In this regard, the flexible elongate member 422 may be sized, shaped, or otherwise configured to be positioned within a body lumen 420 of a patient. In some aspects, the intraluminal device 402 may include a soft atraumatic tip to track the intraluminal device 402 into the body lumen 420. The body lumen 420 may be a blood vessel, such as an artery or a vein of a patient's vascular system, including cardiac vasculature, peripheral vasculature, neural vasculature, renal vasculature; a chamber of the heart; and/or or any other suitable lumen inside the body. For example, the flexible elongate member 422 may be used to direct light into any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. More particularly, the flexible elongate member 422 may be used to direct light adjacent to a site of an infarction. For example, in a cardiac chamber. In addition to natural structures, the flexible elongate member 422 may be used to examine man-made structures such as, but without limitation, heart valves, stents, shunts, filters and other devices.

The flexible elongate member 422 includes a proximal portion and a distal portion. The proximal portion of the flexible elongate member 422 may be coupled to a light controller 413. The proximal portion of the flexible elongate member 422 may be coupled to a light controller 413 via the interface 415, as described further below. For example, in some instances the intraluminal device 402 and/or the flexible elongate member 422 may include and/or be coupled to a connector 414. The connector 414 may be configured to directly or indirectly couple the intraluminal device 402 and/or the flexible elongate member 422 to the interface 415. The interface 415 may couple the intraluminal device 402, the flexible elongate member 422, and/or one or more components of the intraluminal device 402 and/or the flexible elongate member 422 to the light controller 413 and/or the processing system 110. The proximal portion of the flexible elongate member 422 is configured to receive light or signals from the light controller 413. The distal portion of the flexible elongate member 422 is configured to be positioned in contact with the tissue of the patient, i.e. the region of interest, and emit the light controlled by the light controller 413 via the at least one light emitter into tissue of the body lumen 420 of the patient to trigger release of nitric oxide from the tissue and monitor oxygenation of tissue, using the tissue/blood as an absorption source, to identify a level of oxygenation of the tissue. Therefore, in some aspects, the flexible elongate member 422 may extend longitudinally outward from the end of a catheter and positioned in contact with the tissue of the patient. The distal portion of the array includes light emitters and light detectors. In some aspects, the flexible elongate member 422 includes a single light emitter and a single light detector. In some aspects, the flexible elongate member 422 includes a single light emitter and a plurality of light detectors. In some aspects, the flexible elongate member 422 includes a plurality of light emitters and a single light detector. In some aspects, the flexible elongate member 422 includes a plurality of light emitters and a plurality of light detectors. In some aspects, the plurality of light emitters may be a light emitting diode, an optical fiber, etc. In some instances, where the flexible elongate member 422 includes a plurality of light emitters, each of the light emitters in the plurality of light emitters may illuminate several areas of tissue in the body lumen 420 simultaneously. In some instances, where the flexible elongate member 422 includes the plurality of light emitters, each of the light emitters in the plurality of light emitters may be coupled to a same light controller 413. In some instances, where the flexible elongate member 422 includes the plurality of light emitters, one or more of the light emitters in the plurality of light emitters may be coupled to different light controllers, such as multiple light controllers 413. In some instances, when multiple light controllers 413 are utilized, the light controllers 413 may be configured to provide a light the same or different light wavelengths. In some aspects, the light controlled from the light controller 413 may be a wavelength in the ultraviolet light spectrum, the visible light spectrum, or the infrared light spectrum. In some aspects, the light emitted from the light source may be a wavelength of 400 nanometers (nm) to 700 nm, e.g. 670 nm. In some aspects, the light emitted from the light source may be a wavelength of 700 nm to 1 millimeter, e.g. 720 nm. In some instances, the light detectors may be photodiode detectors, photo transistor detectors, photoresistor detectors, etc. The light detectors are configured to monitor an absorption of the light emitted by the light emitter within the tissue of the patient. In some aspects, the absorption of the light within the tissue of the patient indicates a level of oxygenation of the tissue.

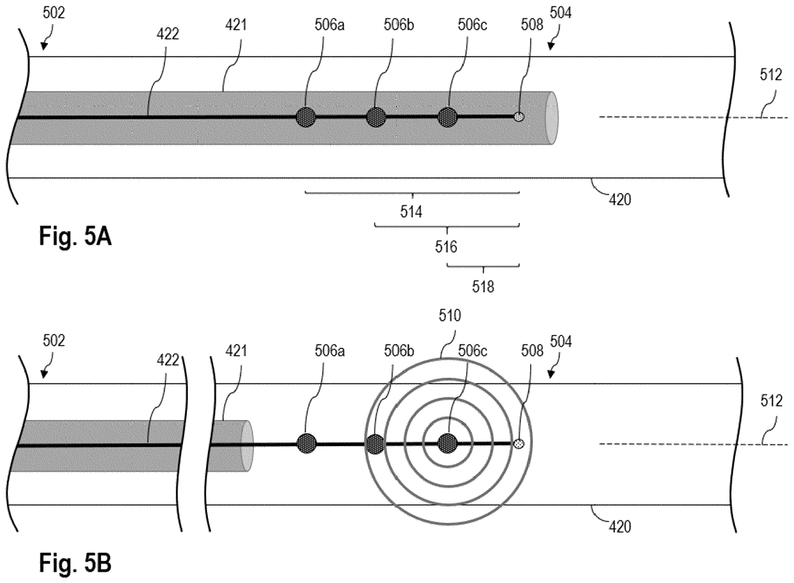

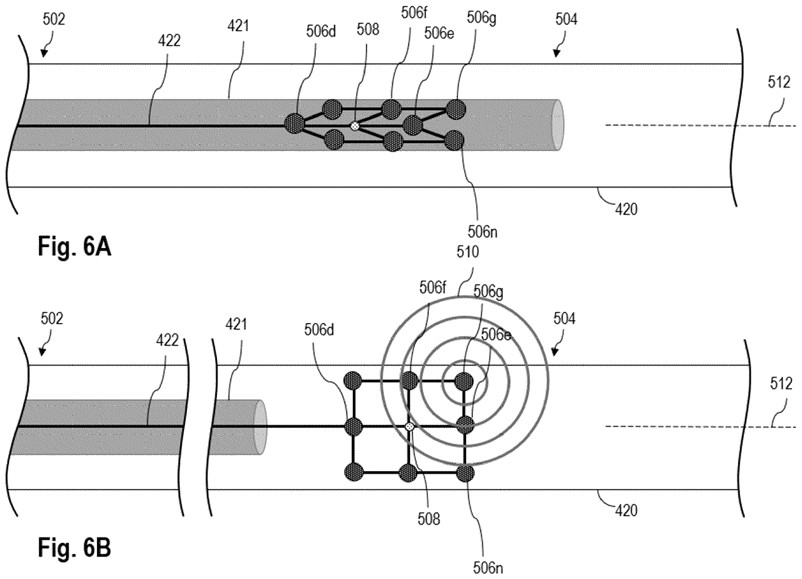

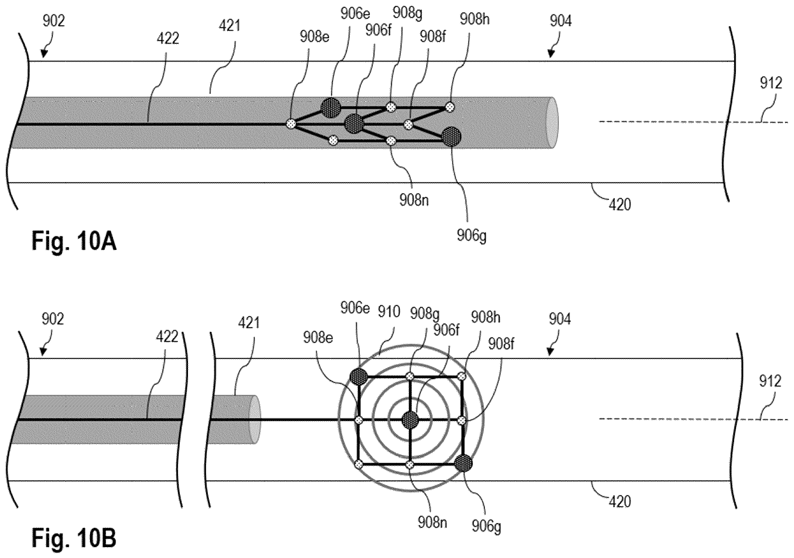

In some aspects, the light emitter is a plurality of light emitters arranged in a linear array. In some instances, one of more of the plurality of light emitters are offset from a light detector in a direction along or parallel to a longitudinal axis of the flexible elongate member 422 (see, e.g., Figs. 5A, 5B, 6A, 6B, 9A, 9B, 10A, and 10B). In some instances, one of more of the plurality of light emitters are offset from a light detector in a direction perpendicular to the longitudinal axis of the flexible elongate member 422 (see, e.g., Figs. 6A, 6B, 10A, and 10B). In some instances, the light emitter is a plurality of light emitters may be arranged in a matrix array, e.g. a 3 x 3 matrix, with the plurality of light emitters on an outside of the matrix and the light detector in the middle of the matrix, as described further below in Fig 6A and 6B.

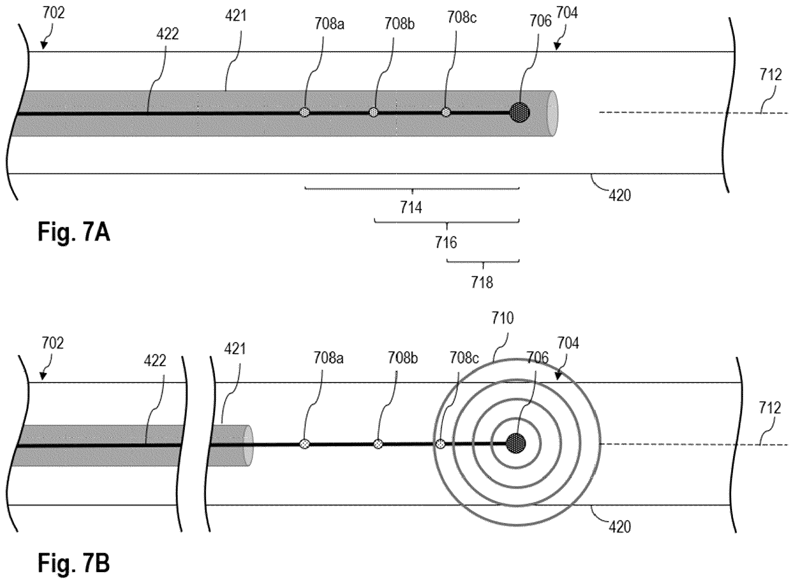

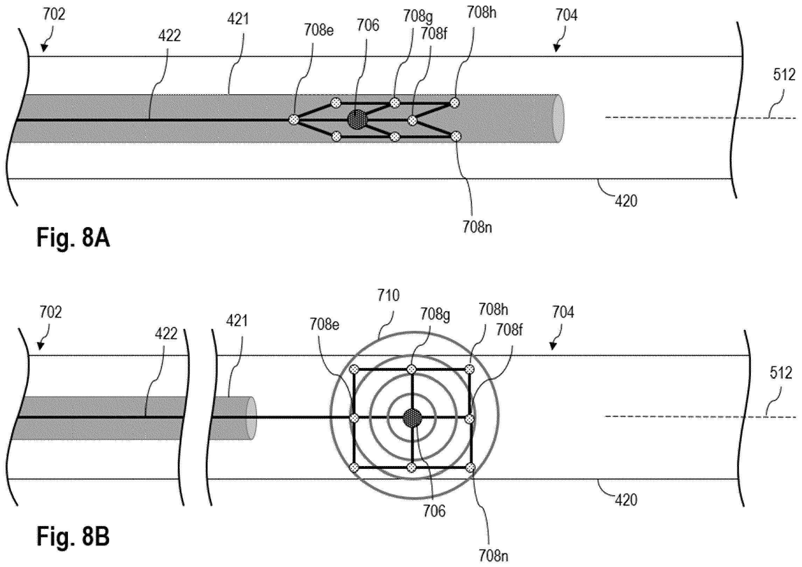

In some aspects, the light detector is a plurality of light detectors arranged in a linear array. In some instances, one or more of the plurality of light detectors are offset from a light emitter along or parallel to a longitudinal axis of the flexible elongate member 422 (see, e.g., Figs. 7A, 7B, 8A, 8B, 9A, 9B, 10A, and 10B). In some instances, one or more of the plurality of light detectors are offset from a light emitter in a direction perpendicular to a longitudinal axis of the flexible elongate member 422 (see, e.g., Figs. 8A, 8B, 10A, and 10B). In some instances, the light detector is a plurality of light detectors arranged in a matrix array, e.g. a 3 x 3 matrix, with the plurality of light detectors on an outside of the matrix and the light emitter in the middle of the matrix, as described further below in Fig 8A and 8B.

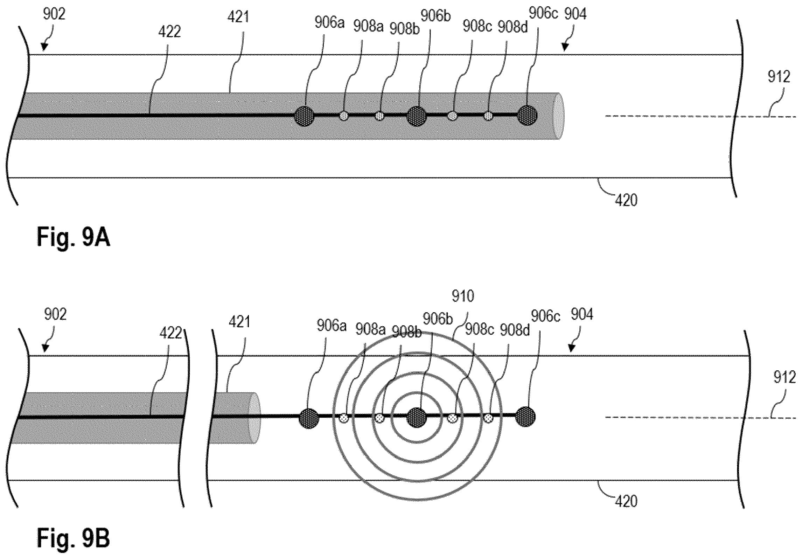

In some aspects, the light emitter is a plurality of light emitters and the light detector is a plurality of light detectors. In some instances, one of more of the plurality of light emitters are offset from one or more of the plurality of light detectors along or parallel to a longitudinal axis of the flexible elongate member 422 (see, e.g., Figs. 9A and 9B). In some instances, one of more of the plurality of light emitters are offset from one or more of the plurality of light detectors in a direction perpendicular to a longitudinal axis of the flexible elongate member 422 (see, e.g., Figs. 10A and 10B). In some instances, one of more of the plurality of light emitters and one or more of the plurality of light detectors are arranged in a matrix array, e.g. a 3 x 3 matrix, as described further below in Fig 10A and 10B.

While some examples of arrangements of one or more light emitters and one or more light detectors are illustrated and described in the present disclosure, it is understood that other orientations and arrangements may be utilized. In this regard, one of more light emitters may be offset from one or more light detectors in directions perpendicular to and/or parallel to a longitudinal axis of the flexible elongate member 422. The one of more light emitters and/or the one or more light detectors may be arranged linearly, in a geometrical pattern, in a spiral pattern, in a matrix pattern, equally spaced, variably spaced, and/or combinations thereof. In this regard, the one of more light emitters and/or the one or more light detectors may be arranged along one or more sides of the flexible elongate member 422 and/or extend circumferentially around (or at least partially around) the flexible elongate member 422.

In each of the various configurations, the light emitter or the plurality of light emitters may be facing the tissue of the patient and configured to emit the light controlled by the light controller 413 into the tissue of the patient. Further, in each of the various configurations, the light detector or the plurality of light detectors, may be facing the tissue of the patient and configured to monitor an absorption of the light within the tissue of the patient. As noted above, the one of more light emitters and/or the one or more light detectors may be arranged along one or more sides of the flexible elongate member 422 and/or extend circumferentially around (or at least partially around) the flexible elongate member 422 such that the light emitted from the light emitters in one or more directions and received by the light detectors in one or more directions. In some instances, one or more light detectors may be positioned to detect the light emitted by one or more of the light emitters. Based on a distance of the offset between a respective light emitter and a respective light detector, a diffusion pattern of scattered light detected by the light detector at the distance provides information about absorption at a depth of the tissue. Thus, in instance where there are a plurality of light detectors and a plurality of light emitters, the distances between respective light detectors and respective light emitters, the diffusion patterns of scattered light detected at different distances can give the user information about absorption at different depths of the tissue.

In some instances, the processing system 110 may be configured to be in communication with the light controller, the light emitter(s), and the light detector(s). In some instances, the processing system 110 may further be configured to control a timing of emission of the light from the light emitter(s) by controlling the light controller 413. In some instances, the processing system 110 may further be configured to control a timing of detection of light by the light detector(s) to monitor the absorption of the light within the tissue of the body lumen 420. Based on the monitored absorption of the light within the tissue of the body lumen 420, the processing system 110 may be configured to output a visual representation of the absorption of the light within the tissue of the body lumen 420 via the display device 120 that is in communication with the processing system 110. In the illustrated example of Fig. 4, the intraluminal device 402 includes a guidewire port 416 and a guidewire lumen 417. In this regard, the intraluminal device 402 may be a rapid-exchange catheter. The guidewire port 416 and the guidewire lumen may allow the intraluminal device 402 to be introduced over a guide wire 418 and into the body lumen 420 of the patient. In some aspects, the intraluminal device 402 includes a guidewire lumen that extends along a majority of a length or the entire length of the intraluminal device 402. In this regard, the intraluminal device 402 may be an over-the-wire catheter. In some instances, the intraluminal device 402 includes one or more lumens 419 that receive flexible elongate member 422 to be positioned within the body lumen 420.

The interface 415 may facilitate communication of signals between the processing system or controller 110, the intraluminal device 402, the flexible elongate member 422, and/or light controller 413. That is, the interface 415 may have appropriate connectors/components for optical, electrical, and/or wireless communication with the processing system or controller 110, the intraluminal device 402, the flexible elongate member 422, and/or light controller 413. In some aspects, the interface 415 and the light controller 413 may be one and the same. That is, the intraluminal device 402 and/or the flexible elongate member 422 may couple directly to the light controller 413, which serves as the interface to the processing system 110, controller, or other component of the intraluminal system 400.

Figs. 5A and 5B are diagrammatic side views of a flexible elongate member 422 within a body lumen 420 according to aspects of the present disclosure. The flexible elongate member 422 includes a proximal portion 502 and a distal portion 504. The proximal portion 502 of the flexible elongate member 422 may be coupled to a light controller 413 and receive light and/or a control signal from the light controller 413. In some aspects, the distal portion 504 of the flexible elongate member 422 is configured to be positioned in contact with the tissue of the patient. In some aspects, the distal portion 504 includes a plurality of light emitters 506a, 506b, and 506c to emit a light once the target tissue is reached, as illustrated by waves 510 in Fig. 5B. The emission of the light from the plurality of light emitters 506a, 506b, and 506c may be controlled by the processing system 110 and/or the light controller 413. The light emitted from the plurality of light emitters 506a, 506b, and 506c may be directed radially outward and into tissue of the body lumen 420. In some aspects, the distal portion 504 includes a light detector 508. The light detector 508 may be configured to monitor an absorption of the light within the tissue of the body lumen 420. As illustrated, the plurality of light emitters 506a, 506b, and 506c are offset from the light detector 508 in a linear array in a direction along or parallel to a longitudinal axis 512 of the flexible elongate member 422. As illustrated, light emitter 506a may be spaced at a distance 514 from light detector 508; light emitter 506b may be spaced at a distance 516, shorter than distance 514, from light detector 508; and light emitter 506c may be spaced at a distance 518, shorter than distances 514 and 516, from light detector 508. Thus, the distances 514, 516, and 518 are all different from one another. In some instances, distance between light emitters 506a, 506b, and 506c may be equal. In some instances, distance between light emitters 506a, 506b, and 506c may be unequal. In some instances, the processing system 110 may be configured to be in communication with the light controller 413, the plurality of light emitters 506a, 506b, and 506c, and the light detector 508. In some instances, the processing system 110 may further be configured to control a timing of emission of the light from the plurality of light emitters 506a, 506b, and 506c by controlling the light controller 413. In some instances, the processing system 110 may further be configured to control a timing of detection of light by the light detector 508 to monitor the absorption of the light within the tissue of the body lumen 420.

Figs. 6A and 6B are diagrammatic side views of a flexible elongate member 422 within a body lumen 420 according to aspects of the present disclosure. Figs. 6A and 6B include features similar to those described in Figs. 5A and 5B. In the instances of Figs. 6A and 6B, the distal portion 504 includes a plurality of light emitters 506d, 506e, 506f, 506g, .., 506n to emit a light once the target tissue is reached, illustrated by waves 510 in Fig. 6B. The emission of the light from the plurality of light emitters 506d, 506e, 506f, 506g, ..., 506n may be controlled by the processing system 110 and/or the light controller 413. The light emitted from the plurality of light emitters 506e-506n may be directed radially outward and into tissue of the body lumen 420. In some aspects, the distal portion 504 includes a light detector 508. The light detector 508 may be configured to monitor an absorption of the light within the tissue of the body lumen 420. In some instances, one of more of the plurality of light emitters, e.g. light emitters 506d and 506e, are offset from the light detector 508 in a direction along or parallel to a longitudinal axis 512 of the flexible elongate member 422. In some instances, one of more of the plurality of light emitters, e.g. light emitters 506f, 506g, .., 506n, are offset from the light detector 508 in a direction perpendicular to the longitudinal axis 512 of the flexible elongate member 422. In some instances, the plurality of light emitters 506d-506n and the light detector 508 may be arranged in a matrix array that may be inserted in a collapsed form, as shown in Fig. 6A, and then fully expanded, as shown in Fig. 6B, once the target tissue area is reached. In some instances, the processing system 110 may be configured to be in communication with the light controller 413, the plurality of light emitters 506e-506n, and the light detector 508. In some instances, the processing system 110 may further be configured to control a timing of emission of the light from the plurality of light emitters 506e-506n by controlling the light controller 413. In some instances, the processing system 110 may further be configured to control a timing of detection of light by the light detector 508 to monitor the absorption of the light within the tissue of the body lumen 420.

Figs. 7A and 7B are diagrammatic side views of a flexible elongate member 422 within a body lumen 420 according to aspects of the present disclosure. The flexible elongate member 422 includes a proximal portion 702 and a distal portion 704. The proximal portion 702 of the flexible elongate member 422 may be coupled to a light controller 413 and receive light and/or a control signal from the light controller 413. In some aspects, the distal portion 704 of the flexible elongate member 422 is configured to be positioned in contact with the tissue of the patient. In some aspects, the distal portion 704 includes a light emitter 706 to emit a light once the target tissue is reached, as illustrated by waves 710 in Fig. 7B. The emission of the light from the light emitter 706 may be controlled by the processing system 110 and/or the light controller 413. The light emitted from the light emitter 706 may be directed radially outward and into tissue of the body lumen 420. In some aspects, the distal portion 704 includes a plurality of light detectors 708a, 708b, and 708c. The plurality of light detectors 708a, 708b, and 708c may be configured to monitor an absorption of the light within the tissue of the body lumen 420. As illustrated, the plurality of light detectors 708a, 708b, and 708c are offset from the light emitter 706 in a linear array in a direction along or parallel to a longitudinal axis 712 of the flexible elongate member 422. As illustrated, light detector 708a may be spaced at a distance 714 from light emitter 706; light detector 708b may be spaced at a distance 716, shorter than distance 714, from light emitter 706; and light detector 708c may be spaced at a distance 718, shorter than distances 714 and 716, from light emitter 706. Thus, the distances 714, 716, and 718 are all different from one another. In some instances, distance between light detectors 708a, 708b, and 708c may be equal. In some instances, distance between light emitters 708a, 708b, and 708c may be unequal. In some instances, the processing system 110 may be configured to be in communication with the light controller 413, the light emitter 706, and the plurality of light detectors 708a, 708b, and 708c. In some instances, the processing system 110 may further be configured to control a timing of emission of the light from the light emitter 706 by controlling the light controller 413. In some instances, the processing system 110 may further be configured to control a timing of detection of light by the light detectors 708a, 708b, and 708c to monitor the absorption of the light within the tissue of the body lumen 420.

Figs. 8A and 8B are diagrammatic side views of a flexible elongate member 422 within a body lumen 420 according to aspects of the present disclosure. Figs. 8A and 8B include features similar to those described in Figs. 7A and 7B. In the instances of Figs. 8A and 8B, the distal portion 704 includes a light emitter 706 to emit a light once the target tissue area is reached, illustrated by waves 710 in Fig 8B. The emission of the light from the light emitter 706 may be controlled by the processing system 110 and/or the light controller 413. The light emitted from the light emitters 706 may be directed radially outward and into tissue of the body lumen 420. In some aspects, the distal portion 704 includes a plurality of light detectors 708e, 708f, 708g, 708h, ..., 708n. The plurality of light detectors 708e, 708f, 708g, 708h, ..., 708n may be configured to monitor an absorption of the light within the tissue of the body lumen 420. In some instances, one of more of the plurality of light detectors, e.g. 708e and 708f, are offset from the light emitter 706 in a direction along or parallel to a longitudinal axis 712 of the flexible elongate member 422. In some instances, one of more of the plurality of light detectors, e.g. light detectors 708g, 708h, ..., 708n, are offset from the light emitter 706 in a direction perpendicular to the longitudinal axis 712 of the flexible elongate member 422. In some instances, the plurality of light detectors 708e-708n and the light emitter 706 may be arranged in a matrix array may that be inserted in a collapsed form, as shown in Fig. 8A, and then fully expanded, as shown in Fig. 8B, once the target tissue area is reached. In some instances, the processing system 110 may be configured to be in communication with the light controller 413, the light emitter 706, and the plurality of light detectors 708e-708n. In some instances, the processing system 110 may further be configured to control a timing of emission of the light from the light emitter 706 by controlling the light controller 413. In some instances, the processing system 110 may further be configured to control a timing of detection of light by the plurality of light detectors 708e-708n to monitor the absorption of the light within the tissue of the body lumen 420.

Figs. 9A and 9B are diagrammatic side views of a flexible elongate member 422 within a body lumen 420 according to aspects of the present disclosure. The flexible elongate member 422 includes a proximal portion 902 and a distal portion 904. The proximal portion 902 of the flexible elongate member 422 may be coupled to a light controller 413 and receive light and/or a control signal from the light controller 413. In some aspects, the distal portion 904 of the flexible elongate member 422 is configured to be positioned in contact with the tissue of the patient. In some aspects, the distal portion 904 includes a plurality of light emitters 906a, 906b, and 906c to emit a light once the target tissue is reached, as illustrated by waves 910 in Fig 9B. The emission of the light from the plurality of light emitters 906a, 906b, and 906c may be controlled by the processing system 110 and/or the light controller 413. The light emitted from the plurality of light emitters 906a, 906b, and 906c may be directed radially outward and into tissue of the body lumen 420. In some aspects, the distal portion 904 includes a plurality of light detectors 908a, 908b, 908c, and 908d that are configured to monitor an absorption of the light within the tissue of the body lumen 420. As illustrated, one or more of the plurality of light detectors 908a, 908b, 908c, and 908d is offset from one or more of the light emitters 906a, 906b, and 906c in a linear array in a direction along or parallel to a longitudinal axis 912 of the flexible elongate member 422. In some instances, the processing system 110 may be configured to be in communication with the light controller 413, the plurality of light emitters 906a, 906b, and 906c, and the plurality of light detectors 908a, 908b, 908c, and 908d. In some instances, the processing system 110 may further be configured to control a timing of emission of the light from the light emitters 906a, 906b, and 906c by controlling the light controller 413. In some instances, the processing system 110 may further be configured to control a timing of detection of light by the light detectors 908a, 908b, 908c, and 908d to monitor the absorption of the light within the tissue of the body lumen 420.

Figs. 10A and 10B are diagrammatic side views of a flexible elongate member 422 within a body lumen 420 according to aspects of the present disclosure. Figs. 10A and 10B include features similar to those described in Figs. 9A and 9B. In the instances of Figs. 10A and 10B, the distal portion 904 includes a plurality of light emitters 906e, 906f, and 906g to emit a light once the target tissue area is reached, illustrated by waves 910 of Fig. 10B, controlled by the processing system 110 and/or the light controller 413. The light emitted from the light emitters 906e, 906f, and 906g may be directed radially outward and into tissue of the body lumen 420. In some aspects, the distal portion 904 is further configured with a plurality of light detector 908e, 908f, 908g, 908h, ..., 908n that are configured to monitor an absorption of the light within the tissue of the body lumen 420. In some instances, one of more of the plurality of light detectors, e.g. 908e and 908f, are offset from a light emitter. e.g. 906f in a direction along or parallel to a longitudinal axis 912 of the flexible elongate member 422. In some instances, one of more of the plurality of light detectors, e.g. light detectors 908g, 908h, ..., 908n, are offset from one or more of the plurality of light emitters, e.g. light emitter 906f, in a direction perpendicular to the longitudinal axis 912 of the flexible elongate member 422. In some instances, the plurality of light emitters 906e-906g and the plurality of light detectors 908e-908n may be arranged in a matrix array that may be inserted in a collapsed form, as shown in Fig. 10A, and then fully expanded, as shown in Fig. 10B, once the target tissue area is reached. In some instances, the processing system 110 may be configured to be in communication with the light controller 413, the plurality of light emitters 906e-906g, and the plurality of light detectors 908e-908n. In some instances, the processing system 110 may further be configured to control a timing of emission of the light from the plurality of light emitters 906e-906g by controlling the light controller 413. In some instances, the processing system 110 may further be configured to control a timing of detection of light by the plurality of light detectors 908e-908n to monitor the absorption of the light within the tissue of the body lumen 420.

In the embodiments disclosed in Figs. 5A, 5B, 6A, 6B, 7A, 7B, 8A, 8B, 9A, 9B, 10A, 10B, reference 421 indicates a flexible elongate sheath through which the flexible elongate member 422 is manipulated. In some embodiments the plurality of light emitters and/or the plurality of light detectors may be arranged in a matrix array that may be inserted in a collapsed form

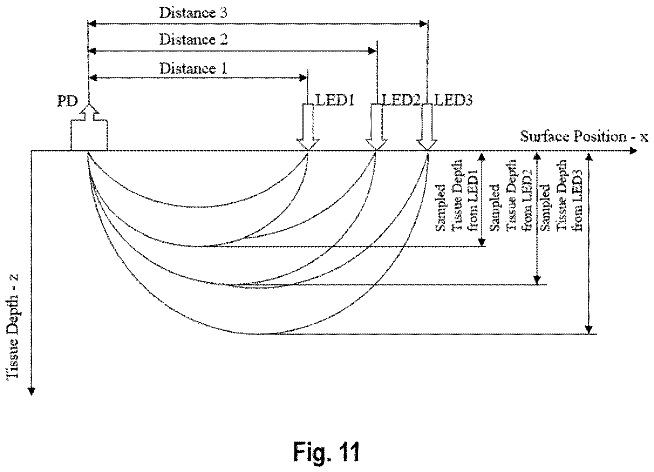

Fig. 11 is an exemplary illustration of resolved depth information from the light emitted from a plurality of light emitters as detected by a light detector according to aspects of the present disclosure. Visible are a plurality of light emitters LED1, LED2, and LED3, which may correlate to light emitters 506c, 506b, and 506a, respectively, of Figs. 5A and 5B, and a light detector PD, which may correlate to light detector 508 of Fig. 5A and 5B, are positioned in contact with the tissue of the patient, indicated by the x axis. While in some instances, LED1, LED2, and LED3, and a light detector PD are aligned horizontally as illustrated in Fig. 11, LED1, LED2, and LED3, and a light detector PD may also be in a aligned vertically, diagonal, or at some other angle. In some aspects, light emitter LED1 is at a distance 1 from the light detector PD, light emitter LED2 is at a distance 2 from the light detector PD, and light emitter LED3 is at a distance 3 from the light detector PD. As light is emitted by each of the each of the light emitters LED1, LED2, and LED3 in a controlled timed sequence, the light detector PD monitors the light detected within the tissue that is indicative of a tissue depth, indicated by the y axis. For example, light detected by light detector PD from light emitter LED1 provides a sampled tissue depth from LED1. As another example, light detected by light detector PD from light emitter LED2 provides a sampled tissue depth from LED2. As still a further example, light detected by light detector PD from light emitter LED3 provides a sampled tissue depth from LED3. The sampled tissue depth from the respective LED in relation to the distance of the LED from the light detector PD provides an indication of the absorption of the light within the tissue of the patient which further indicates a level of oxygenation of the tissue.

Accordingly, it may be seen that the disclosed apparatus advantageously enables the delivery of a 670 nm light to infarcted areas to trigger release stores of nitric oxide from the tissue to increase perfusion to different tissue areas. These nitric oxide stores come from the blood itself and from vessel walls. The nitric oxide is a natural compound in blood vessels that act as a vasodilator, expanding the diameter of blood arteries to allow more blood flow. The dilating effect of nitric oxide increases ability of damaged tissue to repair itself faster with greater influx of oxygenated blood. Further, it may be seen that the disclosed apparatus advantageously enables the monitoring of the delivered light to identify an absorption of the light within the tissue of the patient, which indicates a level of oxygenation of the tissue

The logical operations making up the aspects of the technology described herein are referred to variously as operations, steps, objects, elements, components, or modules. Furthermore, it should be understood that these may be arranged or performed in any order, unless explicitly claimed otherwise or a specific order is inherently necessitated by the claim language. It should further be understood that the described technology may be employed in single-use and multi-use electrical and electronic devices for medical or nonmedical use.

All directional references e.g., upper, lower, inner, outer, upward, downward, left, right, lateral, front, back, top, bottom, above, below, vertical, horizontal, clockwise, counterclockwise, proximal, and distal are only used for identification purposes to aid the reader's understanding of the claimed subject matter, and do not create limitations, particularly as to the position, orientation, or use of the metal ink conductor assembly. Connection references, e.g., attached, coupled, connected, and joined are to be construed broadly and may include intermediate members between a collection of elements and relative movement between elements unless otherwise indicated. As such, connection references do not necessarily imply that two elements are directly connected and in fixed relation to each other. The term "or" shall be interpreted to mean "and/or" rather than "exclusive or." The word "comprising" does not exclude other elements or steps, and the indefinite article "a" or "an" does not exclude a plurality. Unless otherwise noted in the claims, stated values shall be interpreted as illustrative only and shall not be taken to be limiting.

The above specification, examples and data provide a complete description of the structure and use of exemplary aspects of the metal ink conductor assembly as defined in the claims. Although various aspects of the claimed subject matter have been described above with a certain degree of particularity, or with reference to one or more individual aspects, those skilled in the art could make numerous alterations to the disclosed aspects without departing from the spirit or scope of the claimed subject matter.

Still other aspects are contemplated. It is intended that all matter contained in the above description and shown in the accompanying drawings shall be interpreted as illustrative only of particular aspects and not limiting. Changes in detail or structure may be made without departing from the basic elements of the subject matter as defined in the following claims.

## Claims

1. An apparatus (402), comprising:
a flexible elongate member (422) configured to be positioned within a body lumen (420) of a patient;
at least one light emitter (506a) positioned within the flexible elongate member, the at least one light emitter configured to emit light into tissue of the patient; and
at least one light detector (508) positioned within the flexible elongate member, the at least one light detector configured to receive light from the tissue of the patient.

2. The apparatus of claim 1, wherein the received light from the tissue is indicative of an absorption of the light within the tissue.

3. The apparatus of claim 2, wherein the absorption of the light within the tissue of the patient is indicative of a level of oxygenation of the tissue.

4. The apparatus of any of the preceding claims, wherein the at least one light emitter is configured to emit the light having a wavelength of approximately 670-nanometers.

5. The apparatus of any of claims 1 to 4, wherein:
the at least one light emitter (506a) is positioned within a distal portion of the flexible elongate member;
the at least one light detector (508) is positioned within the distal portion of the flexible elongate member; and
the distal portion (504) of the flexible elongate member is configured to be positioned in contact with the tissue of the patient.

6. The apparatus of any of claims 1 to 4, wherein the at least one light emitter includes a plurality of light emitters (506a, 506b) arranged in a linear array.

7. The apparatus of any of claims 1 to 4, wherein the at least one light detector includes a plurality of light detectors (708a, 708b) arranged in a linear array.

8. The apparatus of any of claims 1 to 4, wherein the at least one light emitter includes a plurality of light emitters (506d, 506e, 506f, 506n) arranged in a matrix array and/or the at least one light detector includes a plurality of light detectors (708e, 708f, 708g, 708n) arranged in a matrix array.

9. The apparatus of any of claims 1 to 4,
wherein the at least one light detector is a plurality of light detectors, and
wherein one or more of the plurality of light detectors are offset from the at least one light emitter in a direction along or parallel to a longitudinal axis of the flexible elongate member.

10. The apparatus of any of claims 1 to 4,
wherein the at least one light detector is a plurality of light detectors, and
wherein one or more of the plurality of light detectors are offset from the at least one light emitter in a direction perpendicular to a longitudinal axis of the flexible elongate member.

11. The apparatus of any of claims 1 to 4,
wherein the at least one light emitter is a plurality of light emitters, and
wherein one or more of the plurality of light emitters are offset from the at least one light detector in a direction along or parallel to a longitudinal axis of the flexible elongate member.

12. The apparatus of any of claims 1 to 4,
wherein the at least one light emitter is a plurality of light emitters, and
wherein one or more of the plurality of light emitters are offset from the at least one light detector in a direction perpendicular to a longitudinal axis of the flexible elongate member.

13. A system (400) comprising the apparatus (402) of any of the preceding claims, further comprising:
a processing system (110) configured to be in communication with the at least one light emitter and the at least one light detector,
wherein the processing system is configured to:
control a timing of emission of the light from the at least one light emitter; and
control a timing of detection of light by the at least one light detector, indicative of the absorption of the light within the tissue.

14. The system of claim 13, further comprising:
a display (120) in communication with the processing system,
wherein the processing system is configured to output a visual representation of the absorption of the light within the tissue via the display.

15. A method, comprising:
introducing a flexible elongate member (422) into a body lumen (420) of a patient, wherein the flexible elongate member includes at least one optical fiber;
emitting, by at least one light emitter (506a) positioned within the flexible elongate member, light into tissue of the patient; and
monitoring, by at least one light detector (508) positioned within the flexible elongate member, light received from the tissue of the patient, indicative of an absorption of the light within the tissue.
